# EUROPÄISCHE PATENTSCHRIFT

(11) **EP 3 005 982 B1**
(45) Veröffentlichungstag und Bekanntmachung des Hinweises auf die Patenterteilung: **15.02.2017**
(21) Anmeldenummer: 15186250.5
(22) Anmeldetag: 22.09.2015
(51) Int. Cl.: A61F 2/18, H04R 25/00

(54) **AKTIVES HÖR-IMPLANTAT MIT EINSTELLBARER FIXIERUNG DES AKTOR-ENDSTÜCKS IM MITTELOHR**
ACTIVE HEARING IMPLANT WITH ADJUSTABLE FIXATION OF THE ACTUATOR END PIECE IN THE MIDDLE EAR
IMPLANT AUDITIF ACTIF COMPRENANT UNE FIXATION REGLABLE DE L'EMBOUT D'ACTIONNEUR DANS L'OREILLE MOYENNE

(30) Priorität: 07.10.2014 DE 102014114494
(43) Veröffentlichungstag der Anmeldung: 13.04.2016
(73) Patentinhaber: Heinz Kurz GmbH Medizintechnik, 72144 Dusslingen (DE)
(72) Erfinder: Hüttenbrink, Karl-Bernd, Prof. Dr., 01326 Dresden (DE)
(74) Vertreter: Kohler Schmid Möbus Patentanwälte

(56) Entgegenhaltungen:
- EP-A2- 0 901 779
- DE-U1-202005 015 533

## Beschreibung

Die Erfindung betrifft ein aktives Hör-Implantat mit einer Anordnung zum Einstellen und dauerhaften Fixieren der Relativlage zwischen einem Aktor-Endstück des aktiven Hör-Implantats einerseits und einem oder mehreren umgebenden Knochen im menschlichen Mittelohr andererseits, wobei das Aktor-Endstück eine längs einer Zylinderachse ausgedehnte, insbesondere längliche Form aufweist, und wobei die Anordnung zwei oder mehrere Ankoppelelemente umfasst, vermittels derer das Aktor-Endstück nach seinem operativen Einsetzen in ein menschliches Mittelohr an einem oder mehreren umgebenden Knochen verankert werden kann.

Eine solches aktives Hör-Implantat mit einer Ankopplungsvorrichtung für ein als "Floating Mass Transducer" ausgebildetes Aktor-Endstück ist bekannt etwa aus der DE 20 2005 015 533 U1 oder auch aus der US 2012 / 0 158 135 A1.

Hör-Implantate werden verwendet, um bei ganz oder teilweise fehlenden oder geschädigten Gehörknöchelchen des menschlichen Mittelohrs den auf die Ohrmuschel treffenden Schall bzw. ein entsprechendes Schallsignal zum Innenohr zu übertragen. Man unterscheidet dabei zwischen passiven Gehörknöchelchenprothesen einerseits, die Teile der Gehörknöchelchenkette physisch ersetzen, wobei die Schallleitung "passiv", also ohne Zuhilfenahme von elektronischen Hilfsmitteln erfolgt, und *aktiven* Hör-Implantaten andererseits, die den Schallsignalen entsprechende elektrische Signale aus einem elektronischen Verstärker eines außerhalb des Mittelohrs angebrachten Hörgeräts mittels eines im Mittelohr implantierten Aktors empfangen, dort durch mechanische Bewegung wieder in akustische Schwingungen umsetzen und von einem vibrierenden Aktor-Endstück über geeignete Verbindungselemente in das Innenohr übertragen. Derartige aktive Hör-Implantate und insbesondere die Problematik einer optimalen post-operativen räumlichen Fixierung und Stabilisierung des Aktor-Endstücks im Innenohr sind Gegenstand der vorliegenden Erfindung.

Der Durchmesser des in der Regel zylinderförmigen Aktor-Endstücks ist normalerweise genormt, also immer von gleicher Größe oder zumindest in einer Gruppe bestimmter diskreter Größen zur Auswahl für unterschiedliche individuelle Gegebenheiten beim jeweiligen Patienten vorhanden.

Aus der DE 200 14 659 U1 ist eine Anordnung zum Einstellen und Fixieren der Relativlage zwischen dem Aktor-Endstück ("Aktuator") eines aktiven Hörimplantats einerseits und einem Glied der menschlichen Gehörknöchelchenkette anderseits bekannt. Dabei weist die bekannte Anordnung ein Verbindungselement mit einem ersten Ankoppelelement in Form einer Steckhülse mit Klammern zum Ankoppeln an das Aktor-Endstück, mit einem zweiten Ankoppelelement in Form eines Clips zum Ankoppeln an den Steigbügel sowie mit einem schaftförmigen Mittelteil zwischen dem ersten und dem zweiten Ankoppelelement auf.

In der US-A 5,941,814 wird als erstes Ankoppelelement zum Ankoppeln des Verbindungselements an das Aktor-Endstück eine als hohler Zylinder ausgebildete Crimphülse vorgeschlagen, durch welche das Aktor-Endstück während der Implantationsoperation gesteckt und anschließend mittels eines Crimpwerkzeugs durch Kaltverformung dauerhaft befestigt wird. Zwischen der Außenseite dieser Crimphülse und dem schaftförmigen Mittelteil besteht eine starre mechanische Verbindung, etwa durch einen Schweiß- oder Lötpunkt. Problematisch ist bei dieser bekannten geometrischen Anordnung die fehlende Flexibilität für eine exakte räumliche Relativ-Positionierung zwischen dem vibrierenden Aktor-Endstück und dem ersten Ankoppelelement des Verbindungselements im Mittelohr. So kann zwar durch ein weiteres oder weniger weites Hineinschieben des ersten Ankoppelelements in den zylindrischen Hohlraum der Crimphülse eine gewisse Variabilität in Richtung der Zylinderachse der Crimphülse erreicht werden. Eine Einstellmöglichkeit in einer Richtung senkrecht zu dieser Zylinderachse beseht jedoch überhaupt nicht, so dass die Fein-Positionierung der Verbindungsstelle zwischen dem Aktor-Endstück und dem ersten Ankoppelelement im Raum und damit eine genaue Fixierung der endgültigen Lage der gesamten Anordnung nur höchst ungenau erfolgen kann. Dies führt entsprechend zu ungewünschten Verspannungen innerhalb des Implantat-Aufbaus sowie zu suboptimaler geometrischer Anpassung der Relativlagen der einzelnen Anschlussteile, was letztlich wiederum einen deutlich schlechteren Frequenzgang des gesamten Hör-Implantats und eine geringere Verbesserung der Schallleitung zur Folge hat, als die Gesamtanordnung mit dem aktiven Hörgerät technisch eigentlich leisten könnte.

Eine wesentliche Verbesserung schlägt demgegenüber die DE 10 2010 046 457 B3 vor: Hier ist ein erstes Ankoppelelement geometrisch so gestaltet, dass es das Aktor-Endstück mit zwei gegenüber liegenden parallelen Schenkeln umgreift, die jeweils eine Rastereinrichtung aufweisen, die im Zusammenwirken der beiden Schenkel ermöglicht, das umgriffene Aktor-Endstück auf wählbaren diskreten axialen Positionen in einer Richtung parallel zur Schaftachse des schaftförmigen Mittelteils zu fixieren. Wenn die diskreten axialen Klemmpositionen zwischen den beiden Schenkeln geometrisch fein genug abgestuft sind, lässt sich damit eine äußerst exakte räumliche Einstellung des Ankoppelpunkts zwischen Aktor-Endstück und erstem Ankoppelelement bewirken, welche andererseits auch die Möglichkeit der räumlichen Positionierung der gewünschten Lage des zweiten Ankoppelelements im Mittelohr erheblich verbessert.

Allerdings ist eine solche relativ komplizierte Anordnung schwierig herzustellen und daher kostenintensiv. Bei vielen einfacher aufgebauten, aus dem Stand der Technik bekannten Anordnungen ist ein - etwa als "Floating Mass Transducer" ausgebildetes - Aktor-Endstück einfach mittels der das elektrische Signal von außerhalb des Ohres zuführenden Leitungsdrähte, welche mechanisch und elektrisch mit dem Aktor-Endstück verbunden sind, im Mittelohr "frei schwebend aufgehängt", wie beispielsweise dem aktuellen Prospekt "Vibroplasty^{™} Couplers" der Firma bess medizintechnik GmbH, Berlin, entnehmbar ist. Hierbei ist jedoch keine dauerhaft raumstabile Fixierung des Aktor-Endstücks gewährleistet.

Eine gewisse Abhilfe schafft die Ankopplungsvorrichtung für ein als "Floating Mass Transducer" ausgebildetes Aktor-Endstück gemäß der eingangs zitierten DE 20 2005 015 533 U1, wo eine Verankerung des Aktor-Endstücks nach seinem operativen Einsetzen in ein menschliches Mittelohr an einem oder mehreren umgebenden Knochen mittels seitlich am Floating Mass Transducer angebrachten federelastischen Drähten vorgeschlagen wird. Nachteilig ist hierbei jedoch, dass die gewünschte Stabilität und dauerhafte Verankerung des Aktors mit den dafür vorgesehenen dünnen federelastischen Drähten nicht wirklich zu erzielen ist in Anbetracht der verhältnismäßig großen, durch den freien Raum laufenden Abstände zwischen dem Aktor und den knöchernen Wänden des Mittelohres. Aufgrund der Indikationen zum Einsatz des implantierbaren Hörgerätes, ebenso wie in den zu erwartenden postoperativen Veränderungen wird die Narbenbildung oder die zugrunde liegende Erkrankung eine erhebliche Krafteinwirkung auf jede in das Mittelohr eingebrachte Struktur entstehen lassen, der lange dünne Drähte aufgrund des weiten Abstandes zwischen Aktor und den Wänden des Mittelohres jedenfalls nicht dauerhaft wiederstehen können. Bereits eine geringe Verbiegung der Drähte wird aber zu einer Entkopplung vom Kontaktpunkt des Aktors mit dem Innenohr führen.

Bei der ebenfalls eingangs zitierten US 2012 / 0 158 135 A1 wird der Aktor zwischen Incus und Steigbügelfußplatte gespannt. Sämtliche Stabilisatorelemente sind mechanisch jeweils mit einem vom Aktor bewegten Teil verbunden.

Aufgabe der vorliegenden Erfindung ist es demgegenüber, mit möglichst einfachen technischen Mitteln unaufwändig und kostengünstig eine gattungsgemäße Anordnung der eingangs beschriebenen Art bereit zu stellen, die neben einer bequemen operativen Einpassung des Aktor-Endstücks ins menschliche Mittelohr sowie einer stabilen Ankopplung an den Steigbügel oder gegebenenfalls an ein entsprechendes Verbindungsstück zum Innenohr auch bei seitlich einwirkenden Kräften, wie etwa Narbenzug, Bewegungen bei Luftdruckschwankungen etc. eine dauerhaft exakte Lagefixierung des Aktor-Endstücks gewährleistet, so dass im Endeffekt die vom Aktor erzeugten akustischen Schwingungen in Form von Schallwellen besser an den Steigbügel oder an die zum Innenohr führenden Teile des Hör-Implantats weitergeleitet werden können.

Erfindungsgemäß wird diese Aufgabe auf ebenso überraschend einfache wie wirkungsvolle Weise dadurch gelöst, dass die Anordnung einen Gleitring aufweist, welcher geometrisch so gestaltet ist, dass er über das zylindrische Aktor-Endstück geschoben werden kann und dieses dann in radialer Richtung hält, während der Gleitring in axialer Richtung des Aktor-Endstücks -gegebenenfalls gegen einen Reibungswiderstand-linear verschiebbar bleibt, und dass radial außen am Gleitring zwei oder mehrere radial abstehende Ankoppelelemente zur Verankerung des Gleitrings nach einem operativen Einsetzen der Anordnung in ein menschliches Mittelohr an einem oder mehreren umgebenden Knochen befestigt sind.

Die lockere, ein Gleiten ermöglichende Anbringung des Aktors im erfindungsgemäß vorgesehenen Gleitring stellt eine konstante Positionierung des Aktors mit seinem Koppelelement zum Steigbügel unabhängig von axial einwirkenden Kräften sicher, da der Aktor unproblematisch auf die optimale Position zur Ankopplung an den Steigbügel geschoben werden kann. Die radial abstehenden Ankoppelelemente erlauben zudem die Verankerung in den biologisch vorgeformten, direkt neben dem ovalen Fenster liegenden Knochenöffnungen der Mittelohrmuskeln, die in einem nur geringen Abstand zum Gleitring liegen, so dass nachteilige große Abstände zu den Außenwänden des Mittelohres und daraus resultierende Hebelwirkungen auf die Ankoppelelemente vermieden werden können. Hierdurch wird die Gefahr einer Dislokation durch Narbenzug, Blutungen etc. minimiert.

Besonders bevorzugt und relativ einfach herzustellen sind Ausführungsformen der erfindungsgemäßen Anordnung, bei denen der Gleitring aus Metall und/oder aus Keramik und/oder aus Kunststoff gefertigt ist, um eine möglichst geringe Gleitreibung am Aktor-Endstück zu erzielen.

Um das Einsetzen und die Kontrolle der korrekten Position des Aktor-Endstücks zu erleichtern, ist bei anderen vorteilhaften Ausführungsformen der Erfindung der Gleitring als ein in sich geschlossener Ring oder als ein halb-offener Ring mit einem Schlitz ausgebildet.

Alternativ oder ergänzend zeichnet sich eine bevorzugte Klasse von Ausführungsformen dadurch aus, dass das Aktor-Endstück zylindrisch mit gleichen Querschnitten längs seiner Zylinderachse ausgebildet ist. Damit wird eine optimale Positionierung des Aktor-Endstücks innerhalb des erfindungsgemäß vorgesehenen Gleitrings ermöglicht.

Bei einer dazu alternativen Klasse von Ausführungsformen ist das Aktor-Endstück tonnenförmig mit unterschiedlichen Querschnitten längs seiner Zylinderachse ausgebildet und weist in seinem Mittelbereich eine Ausbauchung in radialer Richtung auf, um bei variabler Positionierung des Gleitrings einen möglichst reibungsarmen Kontakt mit dem Aktor zu ermöglichen.

Vorzugsweise ist bei Ausführungsform des erfindungsgemäßen Hör-Impiantats das Aktor-Endstück als Induktionsspule oder als Piezo-Koppelstange oder als Magnet eines FMT (="Floating Mass Transducer") ausgebildet. Damit können alle -derzeit technisch möglichen-Wirkungsweisen und Aufbauarten eines Aktors durch die Erfindung aufgenommen werden.

Im Hinblick auf eine besonders hohe Variabilität der Anpassung der Verankerung des Gleitrings an die Anatomie des menschlichen Mittelohres ist eine weitere Klasse von Ausführungsformen der erfindungsgemäßen Anordnung zu bevorzugen, bei welcher die radial abstehenden Ankoppelelemente zur Verankerung des Gleitrings stabförmig oder stangenförmig ausgebildet sind.

Alternativ können bei einer Klasse von besonders einfach herstellbaren Ausführungsformen der erfindungsgemäßen Anordnung die radial abstehenden Ankoppelelemente zur Verankerung des Gleitrings aus Draht aufgebaut sein, wobei der Draht eine gewisse Mindestdicke aufweisen sollte, um eine stabile Verankerung auch bei starkem postoperativen Narbenzug oder bei Bewegungen etwa in Folge von Luftdruckschwankungen zu gewährleisten.

Bei einer weiteren Klasse von Ausführungsformen des erfindungsgemäßen Hör-Implantats sind genau zwei gegenüberliegend am Gleitring angeordnete, radial vom Gleitring abstehende Ankoppelelemente vorgesehen, um die Verankerung gegen ein seitliches Abkippen des Gleitrings abzusichern.

Alternativ dazu sind bei einer anderen Klasse von Ausführungsformen der Erfindung mindestens drei, vorzugsweise symmetrisch, um den Umfang des Gleitrings verteilt angeordnete, radial vom Gleitring abstehende Ankoppelelemente vorgesehen, um die Verankerung an möglichst vielen anatomisch variablen Ankopplungspunkten an den Mittelohrwänden vornehmen zu können.

Weitere, ganz besonders bevorzugte Ausführungsformen des erfindungsgemäßen Hör-Implantats zeichnen sich dadurch aus, dass die vom Gleitring wegragenden entfernten Enden der Ankoppelelemente derart ausgebildet sind, dass sie zum Einsetzen und Verankern des aktiven Hör-Implantats in einem menschlichen Mittelohr in natürliche anatomisch vorhandene Öffnungen im umgebenden Mastoid-Knochen, insbesondere in einen jeweiligen Sehnenkanal des Musculus Stapedius und/oder des Musculus Tensor Tympani eingesteckt werden können. Hierdurch wird es dem Otologen ermöglicht, diese -übrigens einzigenanatomisch bereits vorgegebenen und äußerst stabilen natürlichen Verankerungspunkte an den Wänden des menschlichen Mittelohres für eine dauerhaft stabile und exakte Positionierung Aktor-Endstücks zu nutzen.

In der eingangs zitierten DE 20 2005 015 533 U1 ist lediglich angegeben, dass Drähte "im Knochen" verankert werden. Es wird nicht beschrieben, *in welcher Weise* diese Drähte im Knochen verankert werden sollen. Daher muss der Fachmann annehmen, dass es vorgesehen ist, Bohrungen im Knochen einzubringen, denn ohne Bohrung kommt man ja nicht in den Knochen hinein. Die in der DE 20 2005 015 533 U1 offenbarte Idee als solche ist also ohne Bohrungen in die das Mittelohr des Patenten umgebenden Knochen nicht realisierbar. Derartige Bohrungen zur Befestigung von Strukturen im Knochen sind -per se- natürlich schon sehr lange bekannt.

Im Gegensatz dazu liegt bei der vorliegenden Erfindung, wie oben dargelegt, eine wesentliche Neuerung auch darin, eine Nutzung der von der Natur vorgegebenen natürlichen Öffnungen in den Knochenkanälen der Stapediussehne und der Sehne des M. tensotympani zu ermöglichen. Diese natürlichen Öffnungen und Kanäle im Knochen bleiben auch für das weitere Leben des Patienten in seinem Ohr stabil. Bei Vornahme einer Knochenbohrung hingegen kann (und *wird* mit hoher Wahrscheinlichkeit) ein eingesteckter Draht aufgrund der permanenten Umbauvorgänge im Knochen "herumwandern", so dass etwa ein daran befestigter Magnet möglicherweise seiner Halterung beraubt wird. Dies wiederum ist bei den natürlich vorgegebenen Sehnenöffnungen keineswegs zu erwarten. Mit der vorliegenden Erfindung können nun diese Öffnungen als von der Natur vorgegebene Verankerungspunkte, sozusagen als bionische Verankerung im Mittelohr genutzt werden.

Anders als bei der eingangs zitierten US 2012 / 0 158 135 A1 sind beim Hör-Implantat nach der vorliegenden Erfindung die als Stabilisatoren wirkenden Ankoppelelemente nicht an -insbesondere vom Aktorbewegten Teilen verankert, sondern an Aktor-fernen feststehenden Strukturen, nämlich dem umgebenden Mastoid-Knochen.

Um eine Optimierung des Kraftschlusses zwischen Aktor-Endstück und Gleitring bei gleichzeitigem Erhalt der Schwingungsfähigkeit auch bei den intraoperativen Manipulationen während des Einsetzens des Hör-Implantats zu gewährleisten, können die erfindungsgemäße Anordnung oder Teile davon ganz oder teilweise, zumindest im Bereich des Gleitrings und/oder der Ankoppelelemente aus einem Material mit Formgedächtnis, insbesondere aus einer Nickel-Titan-Legierung, vorzugsweise aus Nitinol hergestellt sein. Insbesondere das Material Titan weist neben seiner Festigkeit und ausgezeichneten Schallleitungseigenschaften bekanntermaßen auch eine hervorragende Biokompatibilität am menschlichen Mittelohr auf. Vorteilhaft im Hinblick auf eine postoperative Lageanpassung sind Ausführungsformen der Erfindung, bei denen das Ankoppelelement ganz oder teilweise aus einem Material mit Formgedächtnis (=memory effect) oder superelastischen Eigenschaften, insbesondere aus einer Nickel-Titan-Legierung, vorzugsweise aus Nitinol hergestellt ist, was per se beispielsweise aus der WO 02/069850 A1 oder der US 6,554,861 B2 -allerdings nur im Zusammenhang mit passiven Gehörknöchelchenprothesen- bekannt ist.

Alternativ oder ergänzend können bei weiteren Ausführungsformen Teile der erfindungsgemäßen Anordnung aus einem Keramikmaterial hergestellt sein.

Weitere bevorzugte Ausführungsformen der erfindungsgemäßen Anordnung zeichnen sich dadurch aus, dass am freien, im implantierten Zustand auf den Innenohrbereich gerichteten unteren Ende des Aktor-Endstücks eine Befestigungsvorrichtung angebracht ist, die mittels einer Koppler-Krone am freien unteren Ende des Aktor-Endstücks sowie mit einem Clip oder mit einer -insbesondere geschlitzten- Glocke auf dem Stapes befestigt werden kann. Damit lässt sich eine abrutschsichere, akustisch harte Verbindung des Aktors mit dem Steigbügel als akustischer Eingang in das Innenohr zu gewährleisten.

Falls der natürliche Steigbügel im Mittelohr des Patienten nicht mehr vorhanden oder zu stark deformiert ist, können als Verbindung zum Innenohrbereich etwa als Piston oder als Kugel ausgestaltete künstliche Ankoppelelemente oder auch als Clip oder als Glocke geformte Elemente zur Ankopplung an das -noch ausreichend vorhandene-Steigbügel-Köpfchen oder als Aufnahmeteil für eine Ω-förmige künstliche Steigbügelfiußplatte oder als Stempel zur Ankopplung an die natürliche Steigbügelfußplatte eingesetzt werden. Dazu beschreibt etwa die DE 10 2009 016 468 B3 Ausgestaltungen eines solchen Befestigungselements als Kolben (="Piston"), Stempel oder geschlitzte Glocke. Allerdings handelt es sich bei sämtlichen dieser bekannten Ankoppelelemente jeweils nicht um Teile von gattungsgemäßen aktiven Hör-Implantaten, sondern vielmehr von gattungsfremden passiven Gehörknöchelchenprothesen, die zudem keinerlei Hinweis auf die erfindungsgemäße geometrische Gestaltung des ersten Ankoppelelements des aktiven Hör-Implants zu geben vermögen.

Bei weiteren vorteilhaften Ausführungsformen der Erfindung ist vorgesehen, dass am freien, im implantierten Zustand auf den Trommelfellbereich gerichteten oberen Ende des Aktor-Endstücks eine Trommelfell-Platte aufgebracht ist, die sich im implantierten Zustand gegen das Trommelfell oder ein zwischen Trommelfell und Aktor-Endstück eingebrachtes flächiges Knorpelstück abstützt, um ein Durchwandern/Durchstoßen der Paukenhöhlenabdeckung auch bei späteren Verlagerungen des Trommelfells sicher zu verhindern.

Nachdem das Hör-Implantat operativ im Mittelohr platziert wurde, beginnt die so genannte Einheilphase. In dieser Zeit bilden sich Narben und diese verursachen unvorhersehbar Kräfte, welche dazu führen können, die Prothese aus ihrer lokalen, während der Operation exakt eingestellten Position zu verschieben. Bei einer zu steifen Verbindung mit den Ankoppelelementen kann es außerdem zu erhöhten Druckspitzen kommen, die wiederum zu Beschädigungen des Implantats führen können. Aus diesem Grund ist es sehr hilfreich, wenn dieser Teil des aktiven Hör-Implantats eine gewisse post-operative Mobilität aufweist, so dass er sich postoperativ selbstständig einer veränderten Position in den Randbereichen angleichen kann. Da zudem die anatomischen Gegebenheiten des Ohrs, wie beispielsweise die Lage, die Form und die Größe des Steigbügels, des Ambosses, des Hammers und des Trommelfells variieren, ist es sehr vorteilhaft, wenn Hör-Implantate nicht starr ausgebildet sind, sondern eine gewisse Flexibilität oder Variabilität aufweisen.

Neben der oben beschriebenen postoperativen Positionsverschiebung ergibt sich nach der Implantation von Hör-Implantaten auch noch ein weiteres Problem: Das Mittelohr des menschlichen Körpers stellt nämlich ein "halb offenes Lager " dar. Jedes Implantationsmaterial, welches im Rahmen einer Rekonstruktion des Mittelohres und seiner Strukturen in den Körper eingebracht wird, erfährt dadurch eine besondere Beanspruchung, dass eine kontaminierte und infizierte Umgebung vorherrscht, die in der Regel das Material angreift. Da das Ziel der Implantation eines Hör-Implantats immer auch eine möglichst lange, komplikationsfreie Verweildauer des Implantats im Mittelohr des Patienten sein muss, kann ein lange andauernder Materialangriff zu Beschädigungen des Implantats und/oder zu einer lokalen Infektion führen. Beide Folgen sind nicht tolerabel. Um eine Schädigung sowohl des Implantationsmaterials als auch des umgebenden Gewebes dauerhaft zu verhindern, ist bei einer weiteren besonders bevorzugten Ausführungsform der Erfindung zur Verbesserung der Akzeptanz bei Verringerung der Abstoßungs- und Infektionsgefahr des Implantates zumindest abschnittsweise eine biologisch aktive Beschichtung, insbesondere eine wachstumshemmende und/oder eine wachstumsfördernde und/oder eine antibakteriell wirkende Beschichtung, vorgesehen.

So wird beispielsweise ein direkt ins Innenohr führendes, etwa in Form eines Kolbens beziehungsweise Pistons ausgebildetes, weiteres Befestigungselement eine wachstumshemmende Beschichtung aufweisen, um eine ungewünschte Versteifung und daraus folgende Unbeweglichkeit dauerhaft zu verhindern.

Weitere Merkmale und Vorteile der Erfindung ergeben sich aus der nachfolgenden detaillierten Beschreibung von Ausführungsbeispielen der Erfindung anhand der Figuren der Zeichnung, die erfindungswesentliche Einzelheiten zeigt, sowie aus den Ansprüchen. Die einzelnen Merkmale können je einzeln für sich oder zu mehreren in beliebigen Kombinationen bei Varianten der Erfindung verwirklicht sein.

In der schematischen Zeichnung sind Ausführungsbeispiele der Erfindung dargestellt, welche in der nachfolgenden Beschreibung näher erläutert werden.

Im Einzelnen zeigen:
- Fig. 1a: eine schematische räumliche Darstellung von schräg oben einer Ausführungsform des erfindungsgemäßen aktiven Hör-Implantats mit Trommelfell-Platte einerseits sowie mit Koppler-Krone andererseits am freien Ende des Aktor-Endstücks und mit einer geschlitzten Glocke als Aufnahmeteil für den natürlichen Stapes oder eine Ω-förmige künstliche Steigbügelfußplatte als Verbindungselement zum Innenohr;
- Fig. 1b: die Ausführungsform von Fig. 1a in einer Ansicht schräg von unten;
- Fig. 1c: die Ausführungsform von Fig. 1a in einer Ansicht seitlich schräg von unten;
- Fig. 2a: eine schematische räumliche Darstellung von schräg oben einer Ausführungsform einer einfacheren Ausführungsform des erfindungsgemäßen aktiven Hör-Implantats mit direkter Ankopplung des freien Endes des Aktor-Endstücks an den natürlichen Stapes oder eine Ω-förmige künstliche Steigbügelfußplatte als Verbindungselement zum Innenohr;
- Fig. 2b: die Ausführungsform von Fig. 2a in einer Ansicht schräg von unten; und
- Fig. 2c: die Ausführungsform von Fig. 2a in einer Ansicht seitlich schräg von unten.

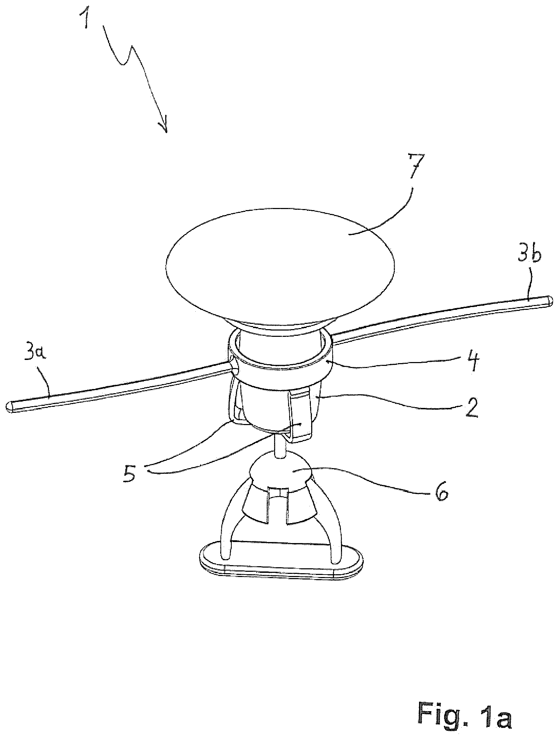

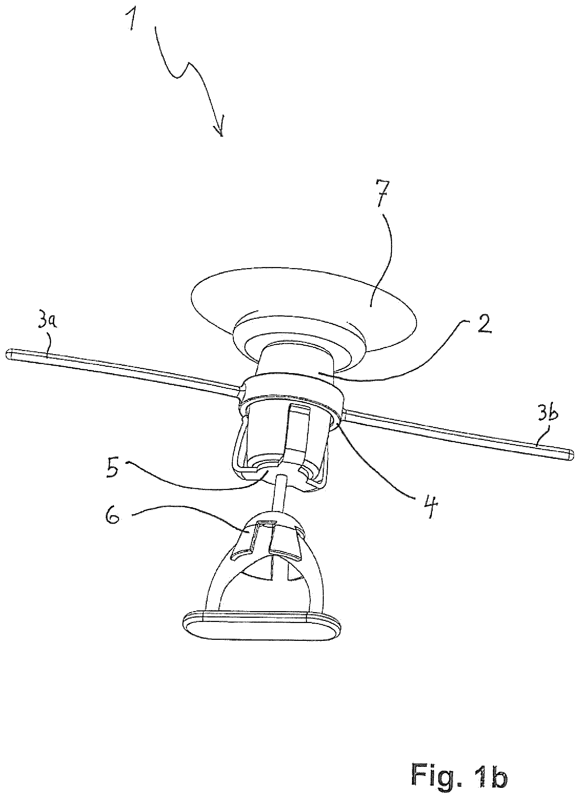

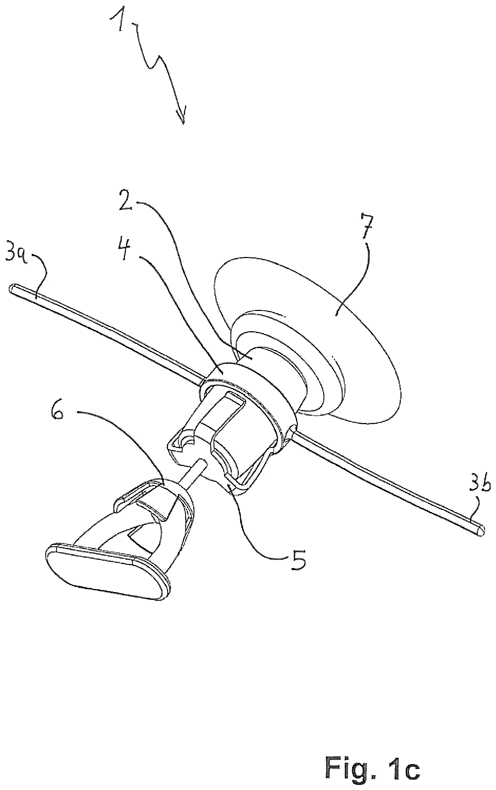

Die in den Figuren 1a bis 2c der Zeichnung schematisch räumlich dargestellten Ausführungsformen der Erfindung weisen jeweils eine **Anordnung 1; 1'** auf, die zum Einstellen und dauerhaften Fixieren der Relativlage zwischen einem **Aktor-Endstück 2** eines - ansonsten in der Zeichnung nicht näher gezeigten - aktiven Hör-Implantats einerseits und einem oder mehreren umgebenden Knochen im menschlichen Mittelohr andererseits. Das Aktor-Endstück 2 weist eine längs einer Zylinderachse ausgedehnte, bei den gezeigten Ausführungsformen längliche Form auf.

Die in der Zeichnung dargestellten Ausführungsformen der Erfindung zeigen zudem jeweils ein leicht tonnenförmig gewölbtes Aktor-Endstück 2 mit unterschiedlichen Querschnitten längs seiner Zylinderachse, welches in seinem Mittelbereich eine -geringe- Ausbauchung in radialer Richtung aufweist. Bei anderen, nicht dargestellten Ausführungsformen ist das Aktor-Endstück zylindrisch mit gleichen Querschnitten längs seiner Zylinderachse ausgebildet.

Das Aktor-Endstück 2 wird in der Regel als Induktionsspule oder als Piezo-Koppelstange oder als Magnet eines FMT (="Floating Mass Transducer") ausgebildet sein.

Die Anordnung 1; 1' umfasst zwei (oder bei in der Zeichnung nicht dargestellten Ausführungsformen auch mehrere) **Ankoppelelemente 3a, 3b,** vermittels derer das Aktor-Endstück 2 nach seinem operativen Einsetzen in ein menschliches Mittelohr an einem oder mehreren umgebenden Knochen verankert werden kann. Die gezeigten Ausführungsformen weisen die radial abstehende Ankoppelelemente 3a, 3b auf, die jeweils stabförmig oder stangenförmig ausgebildet sind. Möglich sind aber auch einfache aus Draht aufgebaute Ankoppelelemente.

Das erfindungsgemäße aktive Hör-Implantat zeichnet sich dadurch aus, dass die Anordnung 1; 1' einen **Gleitring 4** aufweist, welcher geometrisch so gestaltet ist, dass er über das zylindrische Aktor-Endstück 2 geschoben werden kann und dieses dann in radialer Richtung hält, während der Gleitring 4 in axialer Richtung des Aktor-Endstücks 2 -gegebenenfalls gegen einen Reibungswiderstand-- linear verschiebbar bleibt, und dass radial außen am Gleitring 4 zwei oder mehrere radial abstehende Ankoppelelemente 3a, 3b zur Verankerung des Gleitrings 4 nach einem operativen Einsetzen der Anordnung 1; 1' in ein menschliches Mittelohr an einem oder mehreren umgebenden Knochen befestigt sind.

Die Figuren der Zeichnung zeigen Ausführungsformen mit genau zwei gegenüberliegend am Gleitring 4 angeordneten, radial vom Gleitring 4 abstehenden Ankoppelelementen 3a, 3b. Möglich, aber hier nicht dargestellt sind auch Ausführungsformen, bei denen mindestens drei symmetrisch um den Umfang des Gleitrings 4 verteilt angeordnete, radial vom Gleitring 4 abstehende Ankoppelelemente vorgesehen sind.

Die Figuren 1a bis 1c zeigen eine erste, etwas aufwändiger gestaltete Ausführungsform des erfindungsgemäßen aktiven Hör-Implantats mit einer **Koppler-Krone 5** am unteren freien Ende des Aktor-Endstücks 2 und mit einer **geschlitzten Glocke 6** als Aufnahmeteil für den natürlichen Stapes oder eine Ω-förmige künstliche Steigbügelfußplatte als Verbindungselement zum Innenohr. Anstelle der Glocke 6 kann bei in der Zeichnung nicht dargestellten Ausführungsformen als Befestigungselement auch ein Clip vorgesehen sein.

Am oberen Ende des Aktor-Endstücks 2 ist eine **Trommelfell-Platte 7** aufgebracht, die sich im implantierten Zustand gegen das Trommelfell oder ein zwischen Trommelfell und Aktor-Endstück 2 eingebrachtes flächiges Knorpelstück abstützt.

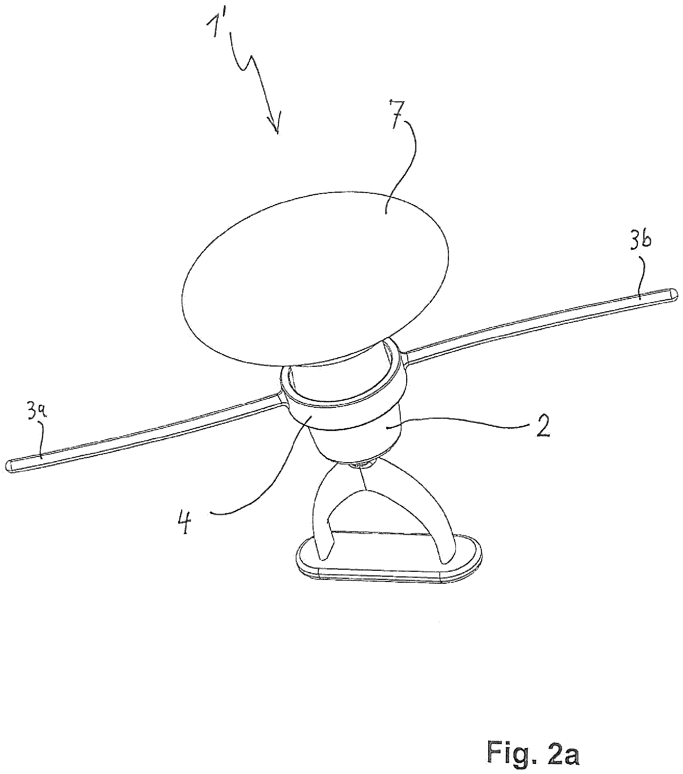

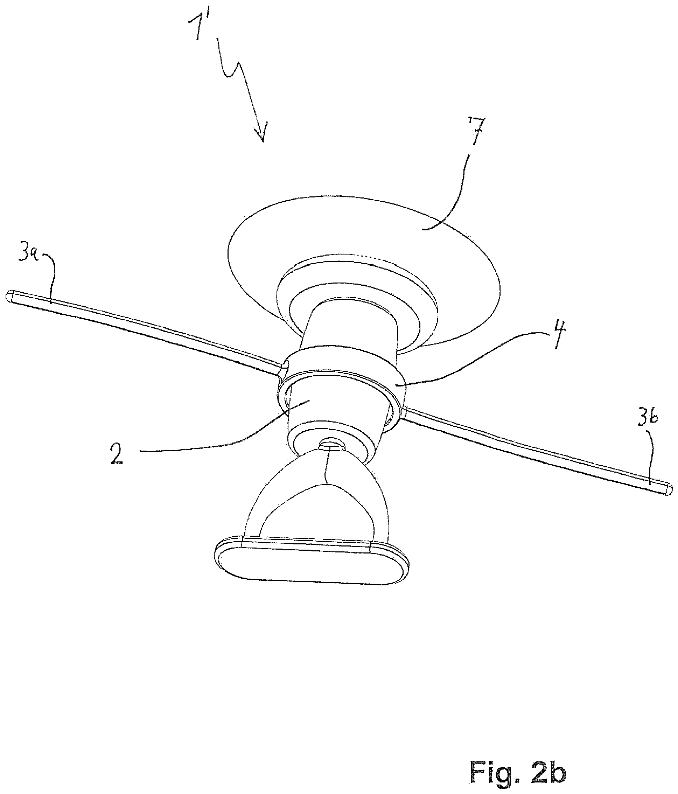

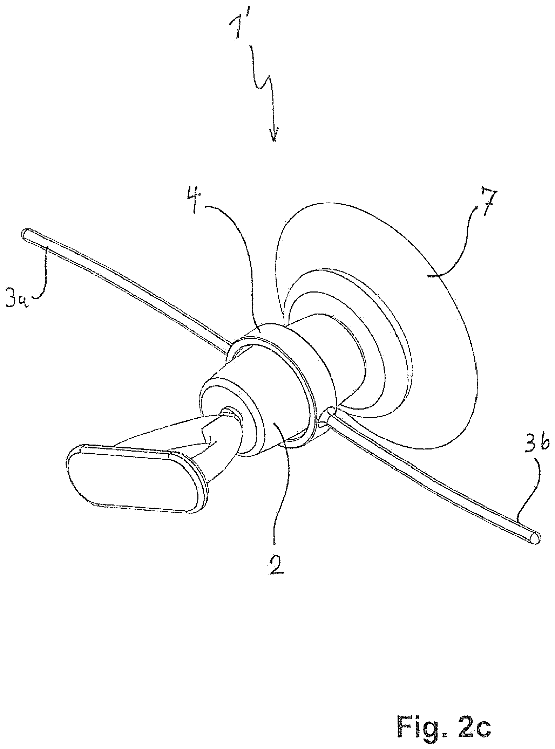

In den Figuren 2a bis 2c hingegen ist eine etwas einfachere Ausführungsform des erfindungsgemäßen aktiven Hör-Implantats dargestellt. Diese zeichnet sich aus durch eine direkte Ankopplung des unteren freien Endes des Aktor-Endstücks 2 an den natürlichen Stapes oder an eine Ω-förmige künstliche Steigbügelfußplatte als Verbindungselement zum Innenohr. Eine Befestigungsvorrichtung wie bei den Ausführungsformen der Figuren 1a bis 1c mit einer Koppler-Krone 5 sowie mit einer Glocke 6 ist bei den einfachen Ausführungsformen der Figuren 2a bis 2c entbehrlich.

Besonders bevorzugt sind die vom Gleitring 4 wegragenden entfernten Enden der Ankoppelelemente 3a, 3b derart ausgebildet, dass sie zum Einsetzen und Verankern des aktiven Hör-Implantats in einem menschlichen Mittelohr in natürliche anatomisch vorhandene Öffnungen im umgebenden Mastoid-Knochen, insbesondere in einen jeweiligen Sehnenkanal des Musculus Stapedius und/oder des Musculus Tensor Tympani eingesteckt werden können.

Die Figuren der Zeichnung zeigen Ausführungsformen der Erfindung, bei denen der Gleitring 4 als ein geschlossener Ring ausgebildet ist. Bei in der Zeichnung nicht näher dargestellten Ausführungsformen der Erfindung kann der Gleitring 4 aber auch als ein halb-offener Ring mit einem Schlitz ausgeführt sein, um höhere radiale Flexibilität zu erzielen.

Ebenfalls in der Zeichnung nicht dargestellt sind Ausführungsformen der erfindungsgemäßen Anordnung 1; 1', bei denen zumindest abschnittsweise eine biologisch aktive Beschichtung, insbesondere eine wachstumshemmende und/oder eine wachstumsfördernde und/oder eine antibakteriell wirkende Beschichtung, vorgesehen ist.

Als Material für die die Anordnung 1; 1' oder Teile davon kommt bevorzugt ein Material mit Formgedächtnis (=memory effect), insbesondere eine Nickel-Titan-Legierung, vorzugsweise Nitinol zum Einsatz. Der Gleitring 4 ist vorzugsweise aus Metall und/oder aus Keramik und/oder aus Kunststoff gefertigt.

## Patentansprüche

1. Aktives Hör-Implantat mit einer Anordnung (1; 1') zum Einstellen und dauerhaften Fixieren der Relativlage zwischen einem Aktor-Endstück (2) des aktiven Hör-Implantats einerseits und einem oder mehreren umgebenden Knochen im menschlichen Mittelohr andererseits, wobei das Aktor-Endstück (2) eine längs einer Zylinderachse ausgedehnte, insbesondere längliche Form aufweist, und wobei die Anordnung (1; 1') zwei oder mehrere Ankoppelelemente (3a, 3b) umfasst, vermittels derer das Aktor-Endstück (2) nach seinem operativen Einsetzen in ein menschliches Mittelohr an einem oder mehreren umgebenden Knochen verankert werden kann,
**dadurch gekennzeichnet,**
**dass** die Anordnung (1; 1') einen Gleitring (4) aufweist, welcher geometrisch so gestaltet ist, dass er über das zylindrische Aktor-Endstück (2) geschoben werden kann und dieses dann in radialer Richtung hält, während der Gleitring (4) in axialer Richtung des Aktor-Endstücks (2) -gegebenenfalls gegen einen Reibungswiderstand- linear verschiebbar bleibt,
und **dass** radial außen am Gleitring (4) zwei oder mehrere radial abstehende Ankoppelelemente (3a, 3b) zur Verankerung des Gleitrings (4) nach einem operativen Einsetzen der Anordnung (1; 1') in ein menschliches Mittelohr an einem oder mehreren umgebenden Knochen befestigt sind.

2. Hör-Implantat nach Anspruch 1, **dadurch gekennzeichnet, dass** der Gleitring (4) aus Metall und/oder aus Keramik und/oder aus Kunststoff gefertigt ist.

3. Hör-Implantat nach Anspruch 1 oder 2, **dadurch gekennzeichnet, dass** der Gleitring (4) als ein geschlossener Ring oder als ein halb-offener Ring mit einem Schlitz ausgebildet ist.

4. Hör-Implantat nach einem der Ansprüche 1 bis 3, **dadurch gekennzeichnet, dass** das Aktor-Endstück zylindrisch mit gleichen Querschnitten längs seiner Zylinderachse ausgebildet ist.

5. Hör-Implantat nach einem der Ansprüche 1 bis 3, **dadurch gekennzeichnet, dass** das Aktor-Endstück (2) tonnenförmig mit unterschiedlichen Querschnitten längs seiner Zylinderachse ausgebildet ist und in seinem Mittelbereich eine Ausbauchung in radialer Richtung aufweist.

6. Hör-Implantat nach einem der vorhergehenden Ansprüche, **dadurch gekennzeichnet, dass** das Aktor-Endstück (2) als Induktionsspule oder als Piezo-Koppelstange oder als Magnet eines FMT (="Floating Mass Transducer") ausgebildet ist.

7. Hör-Implantat nach einem der Ansprüche 1 bis 6, **dadurch gekennzeichnet, dass** die radial abstehenden Ankoppelelemente (3a, 3b) zur Verankerung des Gleitrings (4) stabförmig oder stangenförmig ausgebildet sind.

8. Hör-Implantat nach einem der Ansprüche 1 bis 6, **dadurch gekennzeichnet, dass** die radial abstehenden Ankoppelelemente (3a, 3b) zur Verankerung des Gleitrings (4) aus Draht aufgebaut sind.

9. Hör-Implantat nach einem der Ansprüche 1 bis 8, **dadurch gekennzeichnet, dass** genau zwei gegenüberliegend am Gleitring (4) angeordnete, radial vom Gleitring (4) abstehende Ankoppelelemente (3a, 3b) vorgesehen sind.

10. Hör-Implantat nach einem der Ansprüche 1 bis 8, **dadurch gekennzeichnet, dass** mindestens drei -vorzugsweise symmetrisch- um den Umfang des Gleitrings (4) verteilt angeordnete, radial vom Gleitring (4) abstehende Ankoppelelemente vorgesehen sind.

11. Hör-Implantat nach einem der vorhergehenden Ansprüche, **dadurch gekennzeichnet, dass** die vom Gleitring (4) wegragenden entfernten Enden der Ankoppelelemente (3a, 3b) derart ausgebildet sind, dass sie zum Einsetzen und Verankern des aktiven Hör-Implantats in einem menschlichen Mittelohr in natürliche anatomisch vorhandene Öffnungen im umgebenden Mastoid-Knochen, insbesondere in einen jeweiligen Sehnenkanal des Musculus Stapedius und/oder des Musculus Tensor Tympani eingesteckt werden können.

12. Hör-Implantat nach einem der vorhergehenden Ansprüche, **dadurch gekennzeichnet, dass** am freien, im implantierten Zustand auf den Innenohrbereich gerichteten unteren Ende des Aktor-Endstücks (2) eine Befestigungsvorrichtung angebracht ist, die mittels einer Koppler-Krone (5) am freien unteren Ende des Aktor-Endstücks (2) sowie mit einem Clip oder mit einer, insbesondere geschlitzten, Glocke (6) auf dem Stapes befestigt werden kann.

13. Hör-Implantat nach einem der vorhergehenden Ansprüche, **dadurch gekennzeichnet, dass** am freien, im implantierten Zustand auf den Trommelfellbereich gerichteten oberen Ende des Aktor-Endstücks (2) eine Trommelfell-Platte (7) aufgebracht ist, die sich im implantierten Zustand gegen das Trommelfell oder ein zwischen Trommelfell und Aktor-Endstück (2) eingebrachtes flächiges Knorpelstück abstützt.

14. Hör-Implantat nach einem der vorhergehenden Ansprüche, **dadurch gekennzeichnet, dass** die Anordnung (1; 1') ganz oder teilweise, zumindest im Bereich des Gleitrings (4) und/oder der Ankoppelelemente (3a, 3b) aus einem Material mit Formgedächtnis, insbesondere aus einer Nickel-Titan-Legierung, vorzugsweise aus Nitinol hergestellt ist.

15. Hör-Implantat nach einem der vorhergehenden Ansprüche, **dadurch gekennzeichnet, dass** zumindest abschnittsweise eine biologisch aktive Beschichtung, insbesondere eine wachstumshemmende und/oder eine wachstumsfördernde und/oder eine antibakteriell wirkende Beschichtung, vorgesehen ist.

## Claims

1. Active hearing implant with an arrangement (1; 1') for adjusting and permanently fixing the relative position between an actuator end piece (2) of the active hearing implant on the one hand and one or more surrounding bones in the human middle ear on the other, wherein the actuator end piece (2) has an, in particular elongated, form extending along a cylinder axis, and wherein the arrangement (1; 1') includes two or more coupling elements (3a, 3b) by means of which the actuator end piece (2) can be anchored to one or more surrounding bones after having been surgically inserted in a human middle ear,
**characterised in that**
the arrangement (1; 1') includes a sliding ring (4) which is configured geometrically in such a way that it can be slid over the cylindrical actuator end piece (2) which it then retains in the radial direction while the sliding ring (4) remains linearly displaceable - if appropriate, against a frictional resistance - in the axial direction of the actuator end piece (2), and **in that**, after a surgical insertion of the arrangement (1; 1') in a human middle ear, two or more coupling elements (3a, 3b) projecting radially from the outside of the sliding ring (4) are fastened to one or more surrounding bones.

2. Hearing implant according to Claim 1, **characterised in that** the sliding ring (4) is made of metal and/or ceramic and/or plastics material.

3. Hearing implant according to Claim 1 or 2, **characterised in that** the sliding ring (4) is configured as a closed ring or as a partly open ring with a slot.

4. Hearing implant according to any one of Claims 1 to 3, **characterised in that** the actuator end piece is cylindrical in shape with like cross sections along its cylinder axis.

5. Hearing implant according to any one of Claims 1 to 3, **characterised in that** the actuator end piece (2) is barrel-shaped with varying cross sections along its cylinder axis and has a bulge in the radial direction in its middle region.

6. Hearing implant according to any one of the preceding claims, **characterised in that** the actuator end piece (2) is in the form of an induction coil or a piezoelectric coupling rod or a magnet of an FMT (Floating Mass Transducer).

7. Hearing implant according to any one of Claims 1 to 6, **characterised in that** the radially projecting coupling elements (3a, 3b) for anchoring the sliding ring (4) are in the form of rods or bars.

8. Hearing implant according to any one of Claims 1 to 6, **characterised in that** the radially projecting coupling elements (3a, 3b) for anchoring the sliding ring (4) are built up from wire.

9. Hearing implant according to any one of Claims 1 to 8, **characterised in that** exactly two coupling elements, arranged opposite one another on the sliding ring (4) and projecting radially from the sliding ring (4), are provided.

10. Hearing implant according to any one of Claims 1 to 8, **characterised in that** at least three coupling elements, distributed - preferably symmetrically - around the circumference of the sliding ring (4) and projecting radially from the sliding ring (4), are provided.

11. Hearing implant according to any one of the preceding claims, **characterised in that** the far ends of the coupling elements (3a, 3b) projecting away from the sliding ring (4) are configured in such a way that, for insertion and anchoring of the active hearing implant in a human middle ear, they can be inserted in anatomical openings naturally present in the surrounding mastoid bone, in particular in a respective tendon canal of the stapedial muscle and/or of the tympanic tensor muscle.

12. Hearing implant according to any one of the preceding claims, **characterised in that** a fixing device is attached to the free, in the implanted state lower end of the actuator end piece (2), oriented towards the inner ear region, which fixing device can be fastened to the stapes by means of a coupling crown (5) on the free, lower end of the actuator end piece (2), or with a clip or with an, in particular slotted, bell (6).

13. Hearing implant according to any one of the preceding claims, **characterised in that** a tympanic membrane plate (7) is attached to the free, in the implanted state upper end of the actuator end piece (2), oriented towards the tympanic membrane region, which tympanic membrane plate (7), in the implanted state, rests against the tympanic membrane or against a planar portion of cartilage inserted between tympanic membrane and actuator end piece (2).

14. Hearing implant according to any one of the preceding claims, **characterised in that** the arrangement (1; 1') is produced wholly or partly, at least in the region of the sliding ring (4) and/or of the coupling elements (3a, 3b), from a material having shape memory, in particular from a nickel-titanium alloy, preferably from nitinol.

15. Hearing implant according to any one of the preceding claims, **characterised in that** a biologically active coating, in particular a growth inhibiting and/or a growth promoting and/or an antibacterial coating, is provided, at least in some areas.

## Revendications

1. Implant auditif actif comprenant un agencement (1 ; 1') destiné au réglage et à la fixation permanente de la position relative entre un embout d'actionneur (2) de l'implant auditif actif d'une part, et un ou plusieurs os voisins dans l'oreille moyenne humaine d'autre part, l'embout d'actionneur (2) présentant une forme allongée le long d'un axe cylindrique, en particulier une forme oblongue, et l'agencement (1 ; 1') comprenant deux ou plusieurs éléments de couplage (3a, 3b) au moyen desquels l'embout d'actionneur (2) est susceptible d'être ancré sur un ou plusieurs os voisins, une fois posé par voie chirurgicale dans une oreille moyenne humaine,
**caractérisé en ce que**
l'agencement (1 ; 1') comprend un anneau de glissement (4) qui est conçu géométriquement de manière à pouvoir être poussé par-dessus l'embout d'actionneur (2) cylindrique et à maintenir celui-ci en direction radiale, tandis que l'anneau de glissement (4) reste linéairement mobile en direction axiale de l'embout d'actionneur (2) - le cas échéant à l'encontre d'une résistance de friction -
et **en ce que** deux ou plusieurs éléments de couplage (3a, 3b) dépassant radialement sont fixés radialement à l'extérieur sur l'anneau de glissement (4) en vue d'ancrer l'anneau de glissement (4) sur un ou plusieurs os voisins après une pose chirurgicale de l'agencement (1 ; 1') dans une oreille moyenne humaine.

2. Implant auditif selon la revendication 1, **caractérisé en ce que** l'anneau de glissement (4) est réalisé en métal et/ou en céramique et/ou en matière plastique.

3. Implant auditif selon la revendication 1 ou 2, **caractérisé en ce que** l'anneau de glissement (4) est réalisé en forme d'anneau fermé ou d'anneau semi-ouvert pourvu d'une fente.

4. Implant auditif selon l'une des revendications 1 à 3, **caractérisé en ce que** l'embout d'actionneur est réalisé sous forme cylindrique présentant des sections transversales identiques le long de son axe cylindrique.

5. Implant auditif selon l'une des revendications 1 à 3, **caractérisé en ce que** l'embout d'actionneur (2) est réalisé en forme de tonneau présentant différentes sections transversales le long de son axe cylindrique et comprend un bombement en direction radiale dans sa zone centrale.

6. Implant auditif selon l'une des revendications précédentes, **caractérisé en ce que** l'embout d'actionneur (2) est réalisé sous forme de bobine d'induction ou de barre de couplage piézoélectrique ou d'aimant d'un FMT (= Floating Mass Transducer ou "transducteur à masse flottante").

7. Implant auditif selon l'une des revendications 1 à 6, **caractérisé en ce que** les éléments de couplage (3a, 3b) dépassant radialement et destinés à l'ancrage de l'anneau de glissement (4) sont réalisés en forme de barreau ou de barre.

8. Implant auditif selon l'une des revendications 1 à 6, **caractérisé en ce que** les éléments de couplage (3a, 3b) dépassant radialement et destinés à l'ancrage de l'anneau de glissement (4) sont constitués en fil.

9. Implant auditif selon l'une des revendications 1 à 8, **caractérisé en ce qu'**il est prévu précisément deux éléments de couplage (3a, 3b) agencés à l'opposé sur l'anneau de glissement (4) et dépassant radialement de l'anneau de glissement (4).

10. Implant auditif selon l'une des revendications 1 à 8, **caractérisé en ce qu'**il est prévu au moins trois éléments de couplage dépassant radialement de l'anneau de glissement (4) et agencés en étant répartis de préférence symétriquement autour du pourtour de l'anneau de glissement (4).

11. Implant auditif selon l'une des revendications précédentes, **caractérisé en ce que** les extrémités des éléments de couplage (3a, 3b) éloignées faisant saillie de l'anneau de glissement (4) sont réalisées de manière à pouvoir être insérées dans des ouvertures existant naturellement sur le plan anatomique dans l'os mastoïde voisin, en particulier dans un canal ligamenteux respectif du musculus stapedius et/ou du musculus tensor tympani, afin de poser et d'ancrer l'implant auditif actif dans une oreille moyenne humaine.

12. Implant auditif selon l'une des revendications précédentes, **caractérisé en ce qu'**un dispositif de fixation est agencé à l'extrémité inférieure libre de l'embout d'actionneur (2), dirigée vers la zone de l'oreille interne, dans l'état implanté, dispositif qui est susceptible d'être fixé à l'extrémité inférieure libre de l'embout d'actionneur (2) au moyen d'une couronne de couplage (5) ainsi que sur l'étrier au moyen d'une pince ou d'une cloche (6) en particulier fendue.

13. Implant auditif selon l'une des revendications précédentes, **caractérisé en ce qu'**une plaque de tympan (7) est agencée à l'extrémité supérieure libre de l'embout d'actionneur (2), dirigée vers la zone de tympan, dans l'état implanté, plaque qui, dans l'état implanté, prend appui contre le tympan ou contre un morceau de cartilage surfacique inséré entre le tympan et l'embout d'actionneur (2).

14. Implant auditif selon l'une des revendications précédentes, **caractérisé en ce que** l'agencement (1 ; 1') est réalisé complètement ou partiellement, au moins dans la zone de l'anneau de glissement (4) et/ou des éléments de couplage (3a, 3b), en un matériau à mémoire de forme, en particulier en un alliage de nickel et de titane, de préférence en nitinol.

15. Implant auditif selon l'une des revendications précédentes, **caractérisé en ce qu'**il est prévu au moins par sections un revêtement biologiquement actif, en particulier un revêtement à effet inhibant la croissance et/ou favorisant la croissance et/ou un revêtement à effet antibactérien.
